(19) Europäisches Patentamt
European Patent Office
Office européen des brevets

(11) Publication number: 0 497 313 A1

(12) EUROPEAN PATENT APPLICATION

(21) Application number: 92101468.4

(22) Date of filing: 29.01.92

(51) Int. Cl.5: C07H 17/00, A01N 43/76, A61K 31/70, C12P 19/60

(30) Priority: 31.01.91 JP 11028/91

(43) Date of publication of application: 05.08.92 Bulletin 92/32

(84) Designated Contracting States: DE FR GB IT

(71) Applicant: AJINOMOTO CO., INC.
15-1, Kyobashi 1-chome, Chuo-ku
Tokyo 104(JP)

(72) Inventor: Yamada, Yasuhiro
No. 24-8 Fushiodai 1-chome
Ikeda-shi, Oosaka-fu(JP)
Inventor: Sakuda, Shohei
No. 6-33 Hiyoshidai Ichiban-cho
Takatsuki-shi, Oosaka-fu(JP)
Inventor: Takayama, Seiji, c/o Central
Research Laboratories
Ajinomoto Co., Inc., No. 1-1 Suzuki-cho
Kawasaki-ku, Kawasaki-shi,
Kanagawa-ken(JP)

(74) Representative: Strehl, Schübel-Hopf, Groening
Maximilianstrasse 54 Postfach 22 14 55
W-8000 München 22(DE)

(54) **Allosamidin compounds and a process for production thereof.**

(57) Novel allosamidin compounds, AJI9463A, AJI9463B and AJI9463C represented by the following structural formula and pseudo disaccharide compounds obtained by partial hydrolysis of these compounds with an acid:

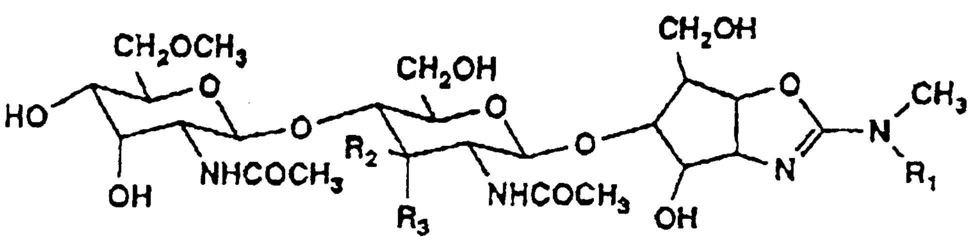

wherein in AJI9463A, $R_1$ and $R_2$ are hydrogen and $R_3$ is hydroxy; in AJI9463B $R_1$ and $R_3$ are hydrogen and $R_2$ is hydroxy; and in AJI9463C $R_1$ is methyl, $R_2$ is hydroxy and $R_3$ is hydrogen.

These compounds exhibit an extremely potent inhibitory activity against the chitinase from Candida albicans and can be utilized as potent antifungal agents and chitinase inhibitors.

EP 0 497 313 A1

## FIELD OF THE INVENTION

The present invention relates to novel allosamidin compounds which are useful in the fields of agricultural chemicals, animal and human drugs, etc. and a process for the production thereof.

## DISCUSSION OF THE BACKGROUND

Chitin is the main component of the cell wall.

While fungi are repeatedly divided and proliferated, the synthesis and decomposition of chitin are well balanced. Secondly, chitin is the principal component found in the exoskeletons of insects. It is known that the synthesis and decomposition of chitin are elaborately controlled also in the process where insects cast the skin and grow.

Chitinase is the major enzyme which participates in the metabolism of chitin. It is thus expected that inhibitors of this enzyme could be a new type of antifungal agents or insect growth controlling agents.

Heretofore there are known 3 substances as chitinase inhibitors: allosamidin (S. Sakuda et al., J. Antibiotics, 40, 296-300, 1987), methylallosamidin (S. Sakuda et al., Preprint of 28Th Discussions and Lectures on Natural Organic Compounds, page 70, 1986) and demethylallosamidin (S. Sakuda et al., Agric. Biol. Chem., 54, 1333-1335, 1990).

Among them, allosamidin and methylallosamidin are compounds found as inhibitors against silkworm-derived chitinase and strongly inhibit endo type chitinase of insects but showed a weak inhibitory activity against fungi-derived chitinase. Demethylallosamidin showed a potent inhibitory activity against chitinase from baker's yeast (Saccharomyces cerevisiae) but its inhibitory activity against chitinase of pathogenic fungi such as Candida albicans is insufficient. It is thus required to develop a more potent chitinase inhibitor.

The three substances described above all have a pseudo trisaccharide structure composed of two 2-amino-2-desoxy-D-allose (N-acetylallosamine molecules and one allosamizoline (or demethylallosamizoline) molecules. N-acetylallosamine is a sugar found for the first time as a constituent of these compounds in the natural world but does not otherwise exist in the natural world. Accordingly, there was a problem to industrially synthesize and supply these compounds.

In view of the actual situation it was considered to be important by the present inventors to investigate allosamidin compounds having a more potent inhibitory activity to chitinase of pathogenic fungi such as Candida albicans, etc. and being more readily synthesized.

Thus, the present inventors searched for such compounds using as an activity index the inhibitory activity to chitinase derived from Candida albicans and as a result, have found that microorganisms belonging to the genus Streptomyces can produce novel allosamidin compounds, AJI9463A, AJI9463B and AJI9463C represented by chemical structure 1 shown below. Based on this finding, the present invention has been accomplished.

## SUMMARY OF THE INVENTION

The present invention relates to allosamidin compounds, AJI9463A, AJI9463B and AJI9463C represented by chemical structure 1 shown below:

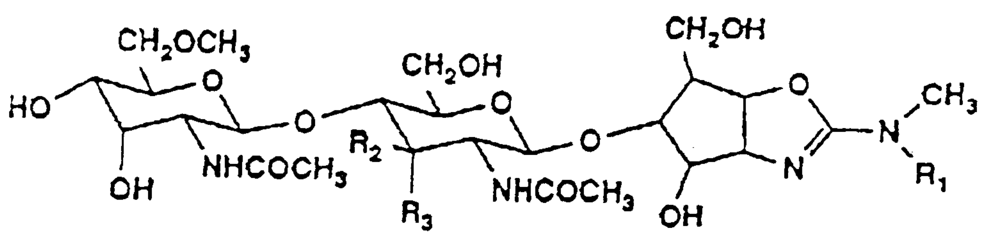

(1)

wherein in AJI9463A, $R_1$ and $R_2$ are hydrogen and $R_3$ is hydroxy; in AJI9463B $R_1$ and $R_3$ are hydrogen and

$R_2$ is hydroxy; and in AJI9463C $R_1$ is methyl, $R_2$ is hydroxy and $R_3$ is hydrogen. It further relates to allosamidin compounds represented by the following chemical structural formula 2 :

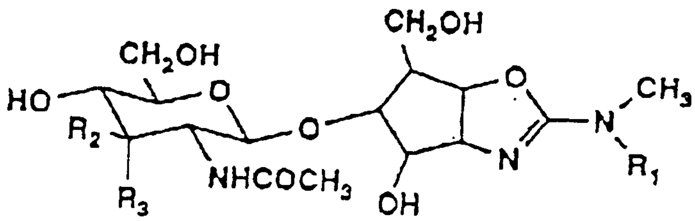

(2)

wherein $R_1$ represents hydrogen or methyl, and when $R_1$ is hydrogen, either $R_2$ or $R_3$ is hydrogen and another is hydroxy; when $R_1$ is methyl, $R_2$ is hydroxy and $R_3$ is hydrogen (hereafter referred to as partially hydrolyzed disaccharides of AJI9463A, AJI9463B and AJI9463C).

The process for preparing the compounds of the present invention, AJI9463A, AJI9463B and AJI9463C, comprises culturing in a suitable medium bacteria capable of producing these compounds, for example, bacteria belonging to the genus Streptomyces, specifically Streptomyces sp. AJ9463 (FERM BP-2801) or Streptomyces sp. AJ9472 (FERM BP- 3705), and harvesting the compounds from the culture broth.

## BRIEF DESCRIPTION OF THE DRAWINGS

Fig. 1 shows proton nuclear magnetic resonance spectrum of AJ9463A.

Fig. 2 shows proton nuclear magnetic resonance spectrum of AJI9463B.

Fig. 3 shows proton nuclear magnetic resonance spectrum of AJI9463C.

Fig. 4 shows proton nuclear magnetic resonance spectrum of the partially hydrolyzed disaccharide of AJI9463C.

## DETAILED DESCRIPTION OF THE PREFERRED EMBODIMENTS

The present inventors have made investigations of inhibitors against fungi-derived chitinase from naturally occurring substances. It has been discovered that allosamidin compounds, AJI9463A, AJI9463B and AJI9463C represented by the following chemical structure 1 produced by a certain allosamidin-producing bacteria, exhibit extremely potent inhibitory activity.

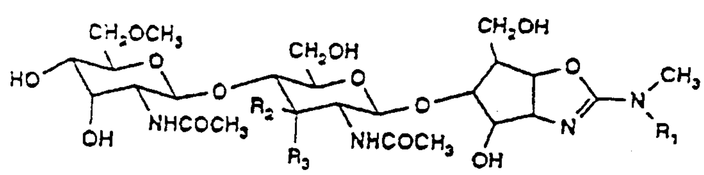

(1)

( In AJI9463A, $R_1$ and $R_2$ are hydrogen and $R_3$ is hydroxy; in AJI9463B $R_1$ and $R_3$ are hydrogen and $R_2$ is hydroxy; and in AJI9463C $R_1$ is methyl, $R_2$ is hydroxy and $R_3$ is hydrogen.

The bacteriological properties of Streptomyces sp. AJ9463 (FERM BP-2801) are as follows.

1. Morphological characteristics:

Under microscopic observation, substrate mycelium is branched on various nutrient agar media and grows well to form well extended aerial mycelium. No verticillate branching is noted. The shape of spore chains is mostly straight or a loose loop. Mature spores are oval and have a size of approximately 1 x (1.5 to 2) µm. The surface is smooth but wrinkled. Spores form chains of 10 to several tens and the boundary

between spores is somewhat unclear. Sporangium, sclerotic granule, motile spore, etc. are not recognized.

2. Growth conditions in various media:

The color hue indication in various media shown below is in accordance with the color standard defined by Japanese Color Research Institute.

(1) Sucrose nitrate agar medium

Moderate growth. Almost colorless substrate mycelium, on which light grey aerial mycelium is formed. Neither remarkable color nor soluble pigment is noted on the back surface.

(2) Glucose asparagine agar medium

Good growth. Aerial mycelium is little noted on light brown substrate mycelium. Neither remarkable color nor soluble pigment is noted on the back surface.

(3) Glycerine asparagine agar medium (ISP5 medium)

Moderate or poor growth. White or grey aerial mycelium is slightly formed on almost colorless substrate mycelium. Neither remarkable color nor soluble pigment is noted on the back surface.

(4) Starch agar medium (ISP4 medium)

Good growth. White or grey or black aerial mycelium is scatteringly formed on greyish red brown substrate mycelium. Neither remarkable color nor soluble pigment is noted on the back surface.

(5) Tyrosine agar medium (ISP7 medium)

Moderate or poor growth. White to grey aerial mycelium is slightly formed on almost colorless substrate mycelium. Neither remarkable color nor soluble pigment is noted on the back surface.

(6) Nutrient agar medium

Good growth. Grey aerial mycelium is abundantly formed on light yellowish brown substrate mycelium. Neither remarkable color nor soluble pigment is noted on the back surface.

(7) Yeast maltose agar medium (ISP2 medium)

Good growth. Grey aerial mycelium is scatteringly formed on light brown substrate mycelium. Neither remarkable color nor soluble pigment is noted on the back surface, but with slightly greyish yellow brown.

(8) Oatmeal agar medium (ISP3 medium)

Good growth. Grey aerial mycelium is abundantly formed on greyish brown substrate mycelium. Neither remarkable color nor soluble pigment is noted on the back surface, but with slightly greyish brown.

3. Physiological characteristics

(1) Growth temperature range:

It grows at 10 to 42 ° C.

(2) Liquefaction of gelatin:

When cultured in glucose peptone gelatin medium at 20 ° C and 27 ° C, liquefaction of gelatin was noted at both temperatures.

(3) Hydrolysis of starch

Positive on starch agar medium (ISP4 medium)

(4) Solidification and peptonization of skimmed milk:

Strong solidification is noted at 37°C but no peptonization is observed. At 27°C, no solidification is observed but strong peptonization is noted.

(5) Formation of melanine-like pigment:

Negative on tyrosine agar medium (ISP7 medium)

(6) Assimilation of carbon sources (Pridham-Gotlieb medium)

good assimilation:
D-glucose, D-xylose, L-rammnose, raffinose
moderate assimilation:
D-fructose, sucrose
no assimilation:
L-arabinose, inositol, D-mannitol
2,6-Diaminopimelic acid contained in the cell wall was LL-type.
From the properties described above, it was revealed that this strain AJ9463 was Streptomyces sp.
The bacteriological properties of Streptomyces sp. AJ9472 (FERM BP- 3705) are as follows.

1. Morphological characteristics

Under microscopic observation, substrate mycelium is branched on various nutrient agar media and grows well to form well extended aerial mycelium. No verticillate branching is noted. The shape of spore chains is mostly straight or loose loop and spore chains are partly branched. Mature spores are oval or spherical and have a size of approximately 0.5 to 0.7 to 2 µm. The surface is smooth. Spores form chains of approximately 20 to 100 and the boundary between spores is somewhat clear . Sporangium, motile spore, etc. are not recognized.

2. Growth conditions in various media:

Color hue indication in various media shown below is in accordance with color standard defined by Japanese Color Research Institute.

(1) Sucrose nitrate agar medium

Poor growth. White to brownish grey aerial mycelium is slightly formed on almost colorless substrate mycelium. Neither remarkable color nor soluble pigment is noted on the back surface.

(2) Glucose asparagine agar medium

Moderate growth. White to brownish white aerial mycelium is formed well on almost colorless substrate mycelium. Neither remarkable color nor soluble pigment is noted on the back surface.

(3) Glycerine asparagine agar medium (ISP5 medium)

Moderate or poor growth. White or brownish white aerial mycelium is slightly formed on almost colorless substrate mycelium Neither remarkable color nor soluble pigment is noted on the back surface.

(4) Starch agar medium (ISP4 medium)

Moderate growth. White or brownish white aerial mycelium is abundantly formed on almost colorless substrate mycelium. Neither remarkable color nor soluble pigment is noted on the back surface.

(5) Tyrosine agar medium (ISP7 medium)

Good or moderate growth. White to brownish white aerial mycelium is moderately formed on almost colorless or yellowish grey substrate mycelium. Neither remarkable color nor soluble pigment is noted on the back surface.

(6) Nutrient agar medium

Good growth. White aerial mycelium is abundantly formed on light yellowish brown substrate mycelium. Neither remarkable color nor soluble pigment is noted on the back surface, but with somewhat light yellowish brown.

(7) Yeast maltose agar medium (ISP2 medium)

Good growth. Brownish white aerial mycelium is abundantly formed on brown substrate mycelium. Light brown is noted on the back surface, and soluble pigment is light brown.

(8) Oatmeal agar medium (ISP3 medium)

Good growth. White or brownish white aerial mycelium is abundantly formed on almost colorless or yellowish grey substrate mycelium. Neither remarkable color nor soluble pigment is noted on the back surface.

3. Physiological characteristics

(1) Growth temperature range:

It grows at 10 to 42 ° C.

(2) Liquefaction of gelatin:

When cultured in glucose peptone gelatin medium at 20 ° C and 27 ° C, liquefaction of gelatin was noted at both temperatures.

(3) Hydrolysis of starch

Negative on starch agar medium (ISP4 medium)

(4) Solidification and peptonization of skimmed milk:

Solidification is negative but peptonization is positive at 27 ° C. Solidification and peptonization are both negative at 37 ° C.

(5) Formation of melanine-like pigment:

Negative on tyrosine agar medium (ISP7 medium)

(6) Assimilation of carbon sources (Pridham-Gotlieb medium: ISP9 medium)

good assimilation:
D-glucose, L-rammnose,
moderate assimilation:
D-xylose, raffinose
no assimilation:
L-arabinose, D-fructose, sucrose, inositol, D-mannitol
2,6-Diaminopimelic acid contained in the cell wall was LL-type.
From the properties described above, it was revealed that this strain AJ9472 was Streptomyces sp.
The culture method is performed in a manner conventionally used to culture ordinary microorganisms

but deep tank culture using a liquid medium is generally advantageous. The medium used to culture may be any medium as long as it contains nutrient sources that can be assimilated by the producing bacteria. That is, as the carbon sources, there may be used glucose, fructose, starch, dextrin, etc.; as the nitrogen sources there may be used meat extract, casein, gluten, yeast extract, soybean powder, corn steep liquor, urea, ammonium sulfate, ammonium phosphate, etc. In addition, there may be used inorganic salts such as sodium hydrogenphosphate, magnesium sulfate, calcium carbonate, etc., if necessary and desired. Upon culture , where foaming is vigorous, defoaming agents such as silicone compounds, higher alcohols, vegetable oils, etc. may also be added in small quantities.

The culture is carried out preferably at 20 to 38 °C, most preferably at about 27°C. The time for the culture may be about 1 to about 10 days but may vary in an appropriate way depending upon conditions for culture.

AJI9463A, AJI9463B and AJI9463C produced by culture are accumulated mainly in the cells. Therefore, these compounds may be generally isolated and purified from the cells separated by means of centrifugation, filtration, etc., using means used for isolation of, e.g., antibiotics. Specifically, means for purification such as solvent extraction with lower alcohols such as methanol, n-butanol, etc.; adsorption column chromatography using silica gel, diatomaceous earth, Avicel, alumina, etc., gel filtration using TOYOPEARL HW40 (carrier for gel filtration manufactured by TOSO Co., Ltd.), etc.; various ion exchange chromatographies and HPLC; additionally countercurrent distribution, crystallization, recrystallization, etc. are used successively or in appropriate combination to isolate and purify these compounds.

The thus isolated and purified allosamidin compounds, AJI9463A, AJI9463B and AJI9463C, have the following physicochemical properties.

(1) AJI9463A

(a) External appearance: white powder
(b) Molecular weight: 622 (FAB MS: m/z 623 $(M + H)^+$, glycerol matrix)
(c) Molecular formula: $C_{25}H_{42}N_4O_{14}$
(d) UV absorption spectrum: terminal absorption in 0.1 N acetic acid
(e) $^1$H-nuclear magnetic resonance spectrum: Fig. 1 (600 MHz, in heavy water containing 3% $CD_3COOD$ or acetic acid-$d_4$
(f) $^{13}$C-nuclear magnetic resonance spectrum: It shows the following signals ($\delta$) at 150 MHz, in heavy water containing 3% $CD_3COOD$ or acetic acid-$d_4$ (provided that this compound takes a plurality of conformations in the solvent and shows a plurality of minor signals considered to be caused by the conformations) 174.6 (Q), 174.3 (Q), 162.4 (Q), 101.2 (CH), 99.9 (CH), 87.6 (CH), 85.5 (CH), 80.8 (CH), 77.5 (CH), 73.2 (CH), 72.7 (CH), 72.0 (CH), 70.6 (CH), 69.7 (CH), 67.1 (CH), 65.0 (CH), 61.5 ($CH_2$), 60.0 ($CH_2$), 59.3 ($CH_3$), 53.4 (CH), 53.2 (CH), 52.3 (CH), 29.0 ($CH_3$), 22.6 ($CH_3$), 22.6 ($CH_3$)

(2) AJI9463B

(a) External appearance: white powder
(b) Molecular weight: 622 (FAB MS: m/z 623 $(M + H)^+$, glycerol matrix)
(c) Molecular formula: $C_{25}H_{42}N_4O_{14}$
(d) UV absorption spectrum: terminal absorption in 0.1 N acetic acid
(e) $^1$H-nuclear magnetic resonance spectrum: Fig. 2 (600 MHz, in heavy water containing 3% $CD_3COOD$ or acetic acid-$d_4$
(f) $^{13}$C-nuclear magnetic resonance spectrum: It shows the following signals ($\delta$) at 150 MHz, in heavy water containing 3% $CD_3COOD$ or acetic acid-$d_4$ (provided that this compound takes a plurality of conformations in the solvent and shows a plurality of minor other signals than those described below which are considered to be caused by the conformations) 175.0 (Q), 174.5 (Q), 162.4 (Q), 101.9 (CH), 100.6 (CH), 87.4 (CH), 85.5 (CH), 80.9 (CH), 80.7 (CH), 75.0 (CH), 73.2 (CH), 72.9 (CH), 72.1 ($CH_2$), 70.6 (CH), 67.3 (CH), 64.9 (CH), 60.9 ($CH_2$), 60.0 ($CH_2$), 59.3 ($CH_3$), 55.5 (CH), 53.3 (CH), 52.2 (CH), 29.0 ($CH_3$), 22.8($CH_3$), 22.6 ($CH_3$)

(3) AJI9463C

(a) External appearance: white powder
(b) Molecular weight: 636 (FAB MS: m/z 637 $(M + H)^+$, glycerol matrix)
(c) Molecular formula: $C_{26}H_{44}N_4O_{14}$

(d) UV absorption spectrum: terminal absorption in 0.1 N acetic acid

(e) $^1$H-nuclear magnetic resonance spectrum: Fig. 3 (400 MHz, in heavy water containing 0.5% $CD_3COOD$ or acetic acid-$d_4$

(f) $^{13}$C-nuclear magnetic resonance spectrum: It shows the following signals (δ) at 100 MHz, in heavy water containing 0.5% $CD_3COOD$ or acetic acid-$d_4$ 175.1 (Q), 174.5 (Q), 161.2 (Q), 102.4 (CH), 100.6 (CH), 87.1 (CH), 85.5 (CH), 81.0 (CH), 80.9 (CH), 75.0 (CH), 73.1 (CH), 72.9 (CH), 72.1 ($CH_2$), 70.6 67.3 (CH), 64.9 (CH), 60.9 ($CH_2$), 59.7 ($CH_2$), 59.3 ($CH_3$), 55.6 (CH), 53.3 (CH), 51.9 (CH), 38.0 ($CH_3$), 22.8 ($CH_3$), 22.6 ($CH_3$)

The structural formulae of these compounds are as described above; among them, AJ9463A is a novel compound in which one of the hydroxy groups at the 6-position of N-acetylallosamidin in demethylallosamidin. It is revealed that this compound shows the most potent inhibitory activity to chitinase derived from Candida albicans as compared to any other compounds found heretofore.

Unlike known allosamidins, AJI9463B and AJI9463C are novel compounds where one of the two N-acetylallosamidins is replaced by N-acetylglucosamine. It is revealed that the inhibitory activity of these compounds against chitinase derived from Candida albicans is comparable to that of N-acetylallosamidin. Furthermore, N-acetylallosamidin is acetylated amino sugar present abundantly in the natural world and is considered advantageous in producing the allosamidins in an industrial scale.

These compounds represented by chemical structural formula 1 can also be subjected to partial hydrolysis under mild conditions using diluted hydrochloric acid, whereby allosamidin compounds represented by the following chemical structural formula 2:

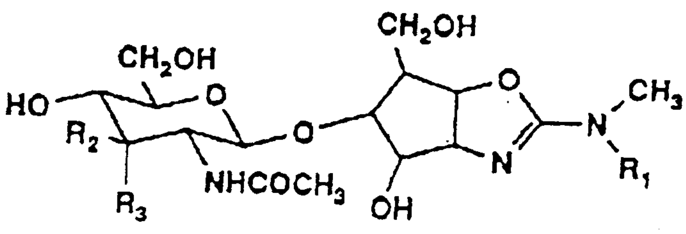

(2)

wherein $R_1$ represents hydrogen or methyl, and when $R_1$ is hydrogen, either $R_2$ or $R_3$ is hydrogen and another is hydroxy; when $R_1$ is methyl, $R_2$ is hydroxy and $R_3$ is hydrogen (hereafter referred to as partially hydrolyzed disaccharides of AJI9463A, AJI9463B and AJI9463C) are formed.

Isolation and purification from the hydrolysates may be effected by appropriately combining the means used for isolation and purification of antibiotics described above.

These compounds represented by chemical structural formula 2 are pseudo disaccharides. It is revealed that these compounds show an inhibitory activity against chitinase derived from Candida albicans which is comparable to the pseudo trisaccharides prior to the partial hydrolysis. In particular, it is expected that the compound of N-acetylglucosamine type in which $R_2$ is hydroxy and $R_3$ is hydrogen would easily be synthesized chemically and is considered to be important from an industrial viewpoint.

That is, allosamizoline is synthesized by, e.g., the method of Trost et al. (J. Amer. Chem. Soc., 112, 1261-1263 (1990)). After protecting the hydroxy groups at the 6- and 3-positions of allosamizoline either with a protective group such as benzoyl or by formation of an acetal such as isopropylidene, sugar such as N-acetylglucosamine, etc. may then be bound to the hydroxy group at the 4-position.

As stated above, the allosamidin compounds represented by chemical structural formula 1 or 2 show a potent inhibitory activity against chitinase derived from Candida albicans. These compounds also affect the growth progress of various fungi including the genus Candida and the genus Fusarium to cause morphological abnormality.

On the other hand, these compounds have low cytotoxicity and showed no growth arrest on mouse ascites breast cancer cells or human leukemia cells even though any of these compound is added in a concentration of 1 mg/ml.

Therefore, by incorporating any of these compounds, the resulting compositions may be utilized as, for example, antifungal agents. The antifungal agents comprising these compounds as the effective ingredient are useful for the treatment of fungal diseases, including human, in which fungi or yeast participates, and for

the treatment of mammal. Fungal growth is inhibited or fungi can be terminated in the living body by preparations such as tablets, capsules or elixir in the case of oral administration; and in the case of parenteral administration, by administering sterile solutions or suspensions. Where the compounds of the present invention are used as the effective ingredient, the compounds may be administered to the patient (animal and human) requiring such treatment in a dose range of 10 to 1000 mg per patient. The dose may vary depending upon condition of disease, body weight of the patient and other factors known to one skilled in the art.

The compounds may be used in the form of physiologically acceptable salts or mixtures thereof, and may be mixed with each other in a unit dose form required for preparing pharmaceutical preparations generally recognized, together with a physiologically acceptable vehicle, carrier, excipient, binder, preservative, stabilizer, flavor, etc. The amount of the active substance in such compositions or preparations is set forth to obtain an appropriate amount indicated.

Specific examples of chemicals which may be admixed to prepare tablets, capsules, etc. include the following compounds: binders such as gum arabic, corn starch or gelatin; excipients such as microcrystalline cellulose; swelling agents such as alginic acid, etc.; lubricants such as magnesium stearate, etc.; dissolution aids such as sodium desoxycholate, etc.; sweeteners such as sucrose, lactose, etc.; flavors such as peppermint, etc.; and in the case of capsules as preparation unit form, further liquid carrier such as oils and fats, in addition to the chemicals described above. Various other materials may be present as coating agents or for the purpose of changing physical shape of preparation unit by another method. For example, tablets may be coated with Shellac or sucrose or with both of them. Syrup or elixir may contain the active compound, sucrose as a sweetener, methyl and propyl parabens as preservatives, pigments and flavors such as cherry or orange flavor.

Sterile compositions for injection may be formulated in a conventional manner for preparing pharmaceutical compositions which comprise dissolving or suspending the active substance in a vehicle such as water for injection, in natural vegetable oil such as sesame oil, palm oil, peanut oil, cotton seed oil, etc. or in a synthetic fat vehicle such as ethyl oleate, etc. A buffer, a preservative, an antioxidant, etc. may also be incorporated, if necessary and desired.

## Examples

### (1) Preparation Example of AJI9463A, AJI9463B and AJI9463C

A medium, pH 7.2, composed of 1.5 g of glucose, 0.5 g of meat extract, 0.2 g of peptone, 0.1 g of yeast extract and 100 ml of water was prepared and charged in a Sakaguchi flask of 500 ml volume. After sterilization at 120°C for 20 minutes, one platinum loop of slant culture cake of *Streptomyces* sp. AJ9463 (FERM BP-2801) was inoculated on the medium and shake cultured at 28°C for 3 days to obtain primary seed mother liquor. On the other hand, a medium having the same composition as above was prepared in a volume of 3 liters and separately charged by 1 liter each in 3 Sakaguchi flasks each having 5 liter volume followed by sterilization at 120°C for 30 minutes. Each flask was charged with 20 ml of the primary seed mother liquor described above. The flasks were shaken at 28°C for 3 days to obtain secondary seed mother liquor. Furthermore, 150 liters of a medium having the same composition as above were prepared and charged in a culture tank followed by sterilization at 120°C for 30 minutes. The tank was charged with 3 liters of the secondary seed mother liquor described above followed by shake culture at 27°C for 66 hours. At this stage, the stirring velocity was 200 rpm and aerial rate was 38 l/min. The thus obtained culture broth was centrifuged by a sharpless centrifuging machine to recover the cells. The wet weight of the thus obtained cells was 2.6 kg.

To the thus obtained cells was added 15 liters of methanol to extract overnight. After Celite was added to the extract, filtration was performed through a funnel to separate the cells from the extract. Subsequently 8 liters of 80% methanol was added to the cells followed by extraction for 3 hours. The cells were separated from the extract through a funnel. The thus obtained extracts were combined and concentrated under reduced pressure. After methanol was removed, distilled water was added to make the whole volume 6 liters.

The whole volume was adsorbed onto a column of Ø5.5 x 49 cm packed with activated carbon. After washing with 2 liters of distilled water, the column was eluted successively with 5 liters of 10% ethanol, 5 liters of 25% ethanol, 5 liters of 50% ethanol and 50% ethanol, whose pH was adjusted to 3.5 with acetic acid. The fraction eluted with 50% ethanol was combined with the fraction eluted with 50% ethanol whose pH was adjusted to 3.5 with acetic acid. The mixture was concentrated under reduced pressure to remove the ethanol. Distilled water was added to the concentrate until the whole volume was 5 liters. At this stage,

the pH of the solution was 3.45.

The solution was passed through a SP-Sephadex C-25 (manufactured by Pharmacia Fine Chemicals, Inc.) column of Ø2 x 54 cm equilibrated with 50 mM ammonium acetate (pH 5.0) and then eluted with 50 mM ammonium acetate (pH 5.0). The active fraction was recovered and lyophilized.

The lyophilized product was dissolved in 30 ml of 0.1 N acetic acid and then purified by high performance liquid chromatography using Capcell Pak ODS Column (Capcell Pak $C_{18}$ SG120 column; Ø20 x 250 mm, particle diameter of the packing agent, 5 µm, manufactured by SHISEIDO Co., Ltd.). The aforesaid 0.1 N acetic acid solution was poured in 500 µl portions per time and the procedure was repeated for fractionation. Elution was performed by a linear gradient in which the acetonitrile concentration in 10 mM ammonium acetate aqueous solution (pH 8.9) was increased. For the initial 2 minutes, elution was performed with 10 mM ammonium acetate and thereafter the acetonitrile concentration was linearly increased up to 40%. In this case, the flow rate was 5 ml/min. Each active component was detected by UV absorption at 220 nm. Elution time of each known component under the elution conditions described above was: 28.0 minutes in demethylallosamidin, 37.6 minutes in allosamidin and 41.2 minutes in methylallosamidin. In this case, elution time was 32.6 minutes, 31.2 minutes and 39.1 minutes in AJI9463A, AJI9463B and AJI9463C, respectively.

Each component was fractionated and concentrated under reduced pressure. Each concentrate was then lyophilized. The yields of AJI9463A, AJI9463B and AJI9463C were 0.4 mg, 1.2 mg and 2.1 mg, respectively.

(2) Preparation Example of AJI9463A, AJI9463B and AJI9463C

A medium, pH 7.2, composed of 1.5 g of glucose, 0.5 g of meat extract, 0.2 g of peptone, 0.1 g of yeast extract and 100 ml of water was prepared and charged in a Sakaguchi flask of 500 ml volume. After sterilization at 120°C for 20 minutes, one platinum loop of slant culture cake of Streptomyces sp. AJ9472 (FERM BP-3705) was inoculated on the medium and shake cultured at 28°C for 3 days to obtain primary seed mother liquor. On the other hand, a medium having the same composition as above was prepared in a volume of 4 liters and separately charged by 1 liter each in 4 Sakaguchi flasks each having 5 liter volume followed by sterilization at 120°C for 30 minutes. Each flask was charged with 20 ml of the primary seed mother liquor described above. The flasks were shaken at 28°C for 3 days to obtain the secondary seed mother liquor. Furthermore, 200 liters of medium having the same composition as above were prepared and charged in a culture tank followed by sterilization at 120°C for 30 minutes. The tank was charged 4 liters of the secondary seed mother liquor described above followed by shake culture at 27°C for 66 hours. At this stage, a stirring velocity was 200 rpm and aerial rate was 50 l/min. The thus obtained culture broth was centrifuged by a sharpless centrifuging machine to recover the cells. The wet weight of the thus obtained cells was 4.5 kg.

To the thus obtained cells was added 20 liters of methanol to extract overnight. After cellite was added to the extract, filtration was performed through a funnel to separate the cells from the extract. Subsequently 8 liters of 80% methanol was added to the cells followed by extraction for 3 hours. The cells were separated from the extract through a funnel. The thus obtained extracts were combined and concentrated under reduced pressure. After methanol was removed, distilled water was added to make the whole volume 8 liters.

The whole volume was adsorbed onto a column of Ø6 x 50 cm packed with activated carbon. After washing with 2.4 liters of distilled water, the column was eluted successively with 7 liters of 10% ethanol, 7 liters of 25% ethanol, 7 liters of 50% ethanol and 50% ethanol whose pH was adjusted to 3.5 with acetic acid. The fraction eluted with 50% ethanol was combined with the fraction eluted with 50% ethanol whose pH was adjusted to 3.5 with acetic acid. The mixture was concentrated under reduced pressure to remove the ethanol. Distilled water was added to the concentrate until the whole volume was 7 liters. At this stage, the pH of the solution was 3.45.

The solution was passed through a SP-Sephadex C-25 (manufactured by Pharmacia Fine Chemicals, Inc.) column of Ø2.4 x 60 cm equilibrated with 50 mM ammonium acetate (pH 5.0) and then eluted with 50 mM ammonium acetate (pH 5.0). The active fraction was recovered and lyophilized.

The lyophilized product was dissolved in 40 ml of 0.1 N acetic acid and then purified by high performance liquid chromatography using Capcell Pak ODS Column (Capcell Pak $C_{18}$ SG120 column; Ø20 x 250 mm, particle diameter of the packing agent, 5 µm, manufactured by SHISEIDO Co., Ltd.). The aforesaid 0.1 N acetic acid solution was poured in 500 µl portions per time and the procedure was repeated for fractionation. Elution was performed by a linear gradient in which the acetonitrile concentration in 10 mM ammonium acetate aqueous solution (pH 8.9) was increased. For the initial 2 minutes, elution was

performed with 10 mM ammonium acetate and thereafter the acetonitrile concentration was linearly increased up to 40%. In this case, the flow rate was 5 ml/min. Each active component was detected by UV absorption at 220 nm. Elution time of each known component under the elution conditions described above was: 28.0 minutes in demethylallosamidin, 37.6 minutes in allosamidin and 41.2 minutes in methylallosamidin. In this case, elution time was 32.6 minutes, 31.2 minutes and 39.1 minutes in AJI9463A, AJI9463B and AJI9463C, respectively.

Each component was fractionated and concentrated under reduced pressure. Each concentrate was then lyophilized. The yields of AJI9463A, AJI9463B and AJI9463C were 10.7 mg, 70.6 mg and 121.3 mg, respectively.

(3) Preparation of partially hydrolyzed disaccharide of AJI9463A, AJI9463B and AJI9463C

In this embodiment, hydrolysis of AJI9463C is given as an example but other components can be prepared in a similar way.

AJI9463C, 12.6 mg, was charged in an eggplant flask of 20 ml volume and dissolved in 3 ml of 0.5 N hydrochloric acid. After sealing, the flask was heated at 70°C for 390 minutes on an oil bath. The reaction solution was purified by high performance liquid chromatography using Capcell Pak ODS column shown in Example (1) described above. The conditions for elution were the same as those described in Example (1) above. The elution time of the partial hydrolysate of AJI9463C was 32.8 minutes under the conditions.

The thus isolated and purified partially hydrolyzed disaccharide of AJI9463C (corresponding to the compound having chemical structural formula 2, wherein $R_1$ is methyl, $R_2$ is hydroxy and $R_3$ is hydrogen) has the following physicochemical properties.

(a) External appearance: white powder
(b) Molecular weight: 419 (FAB MS: m/z 420 $(M + H)^+$, glycerol matrix)
(c) Molecular formula: $C_{17}H_{29}N_3O_9$
(d) UV absorption spectrum: terminal absorption in 0.1 N acetic acid
(e) $^1$H-nuclear magnetic resonance spectrum: Fig. 4 (400 MHz, in heavy water containing 0.5% )

(4) Assay for chitinase inhibitory activity by AJI9463A, AJI9463B and AJI9463C and the partially hydrolyzed disaccharides thereof

(i) Preparation of chitinase solution

The chitinase from Candida albicans (C. albicans) was prepared by the following method. Firstly, Sabouraud medium (pH 5.6) composed of 4 g of glucose, 1 g of polypeptone and 100 ml of water was prepared and charged in an Erlenmeyer flask of 500 ml volume. After sterilization at 120°C for 20 minutes, one platinum loop of slant culture cells of C. albicans ATCC 10231 was inoculated on the medium and then shake cultured at 37°C for 2 days to obtain the primary seed mother liquor. On the other hand, 1 liter of a medium having the same composition as above was prepared and separately charged in portion of 100 ml in 10 Erlenmeyer flasks each having 500 ml volume followed by sterilization at 120°C for 20 minutes. Each flask was charged with 2 ml of the primary seed mother liquor described above. The flasks were shaken at 37°C for 2 days to obtain the secondary seed mother liquor. Furthermore, 50 liters of a medium having the same composition as described above were prepared and charged in a culture tank of 100 liter volume followed by sterilization at 120°C for 30 minutes. The tank was charged with 1 liter of the secondary seed mother liquor described above followed by shake culture at 37°C for 15 hours. At this stage,the stirring velocity was 140 rpm and the aerial rate was 100 l/min. The thus obtained culture broth was centrifuged by a sharpless centrifuging machine to recover the cells. The wet weight of the thus obtained cells was 250 g.

The thus obtained cells were suspended in 800 ml of a buffer for cell homogenization (50 mM bis-Tris, 0.25 M saccharose, 1 mM disodium ethylenediaminetetraacetate, pH 6.5). The suspension was passed through a Dyno-mill disintegrator (beads: MK-2GX, 0.25-0.5 mm) twice to disrupt the cells and the supernatant was recovered. The supernatant was centrifuged at 9000 g for 30 minutes to recover the supernatant. The supernatant was further super-centrifuged at 152000 g for an hour to recover the supernatant. The supernatant was concentrated to about one-fifth of the volume by ultrafiltration (Toyo Ultrafilter UP-20) having the excluding limiting molecular weight of 20,000. The concentrate was used as the crude chitinase solution from Candida albicans in the following assay.

The chitinase from baker's yeast (S. cerevisiae) was prepared by the following method. Firstly, 400 g of baker's yeast (manufactured by Kanegafuchi Chemical Industry Co., Ltd.) was suspended in buffer for enzyme extraction (0.1% digitonin: manufactured by Wako Pure Chemical Industry Co., Ltd.), 0.1% β-mercaptoethanol and 2 liters of 25 mM MES (manufactured by Nakarai Chemical Co., Ltd.) followed by shaking at 30 °C for 2 hours at 120 spm to extract the enzyme. The extract was centrifuged at 12000 g for 10 minutes and the supernatant was recovered. The supernatant was concentrated to about one-fifth of the volume by ultrafiltration (UP-20). After 800 ml of sodium citrate buffer (which was obtained by adding 0.15 M sodium citrate aqueous solution to 0.15 M citric acid aqueous solution and adjusting the pH to 3.0) was added to the concentrate, the formed precipitates were removed by centrifugation (0 °C, 12000 g, 10 minutes). The supernatant was again concentrated by ultrafiltration (UP-20) to about 80 ml. The concentrate was used as the chitinase from baker's yeast in the following assay.

The chitinase from Trichoderma sp. was provided in the following assay after diluting commercially available Chitinase T-I (manufactured by Asahi Industry Co., Ltd.) with McILvanine buffer (obtained by adding 0.2 M disodium hydrogenphosphate aqueous solution to 0.1 M citric acid aqueous solution and adjusting the pH to 5.2) to a concentration of 50 µg/ml.

(ii) Assay for chitinase inhibitory activity

In a vial bottle (volume of 20 ml) were charged 50 µl of the chitinase solution from Candida albicans prepared as described above, 50 µl of 100 µg/ml of 4-methylumbelliferyl β-D-N,N',N''-triacetylchitotrioside (hereinafter abbreviated as 4MBTC, SIGMA Co.) aqueous solution, 75 µl of 50 mM bis-Tris buffer (pH 6.5) and 25 µl of 0.1 N acetic acid. The reaction was carried out at 37 °C for 30 minutes. As a blank, the reaction was carried out in a similar manner using 50 mM bis-Tris buffer (pH 6.5) instead of 50 µl of the enzyme solution. After the reaction, 0.5 M glycine-sodium hydroxide buffer (pH 10.4) was added to make the whole volume 5 ml. Then, the fluorescent intensity was determined with a fluorophotometer (manufactured by Hitachi Ltd., MPF-4). The excited wavelength was 350 nm and fluorescent wavelength was 440 nm. The difference in the fluorescent intensity between the read-out data when the enzyme was added and blank read-out data was made chitinase activity.

In determining the inhibitory activity of AJI9463A, AJI9463B and AJI9463C and the partially hydrolyzed disaccharides thereof, each compound was dissolved in 0.1 N acetic acid and 25 µl of the solution was added in place of 25 µl of 0.1 N acetic acid in the reaction system described above. Further in determining the inhibitory activity against chitinase of baker's yeast and Trichoderma sp. , the assay was performed by changing the buffer added in the reaction system described above from 50 mM bis-Tris buffer (pH 6.5) to 0.1 M citrate buffer (pH 3.0) to McILvanine buffer (pH 5.2), respectively.

(iii) Results

The enzyme inhibitory activity ($IC_{50}$: µg/ml) of AJI9463A, AJI9463B and AJI9463C and the partially hydrolyzed disaccharides thereof against the chitinase from Candida albicans, baker's yeast and Trichoderma sp. is shown in Table 1 described below.

Table 1

| Compound | Chitinase Inhibitory Activity $IC_{50}$ (µg/ml) | | |
|---|---|---|---|
| | C. albicans | S. cerevisiae | Trichoderma sp. |
| AJI9463A | 0.6 | 0.4 | 1.3 |
| AJI9463B | 0.8 | 0.5 | 1.6 |
| AJI9463C | 3.4 | 31.3 | 0.8 |
| Partially hydrolyzed disaccharide of AJI9463A | 0.9 | >200.0 | >50.0 |
| Partially hydrolyzed disaccharide of AJI9463B | 1.0 | >200.0 | >50.0 |
| Partially hydrolyzed disaccharide of AJI9463C | 1.3 | >200.0 | >50.0 |
| Allosamidin | 6.2 | 33.8 | 0.8 |
| Methylallosamidin | 8.8 | 37.2 | 1.2 |

(5) Effects of AJI9463A, AJI9463B and AJI9463C and the partially hydrolyzed disaccharides thereof on growth of Candida albicans

Candida albicans (ATCC 10231) was precultured in a Sabouraud dextrose medium and inoculated on a medium containing 50 µg/ml each of AJI9463A, AJI9463B and AJI9463C and the partially hydrolyzed disaccharides thereof and a medium containing none of them in a definite amount. The change with time after subsequent culture was observed. The observation was microscopically made by sampling the medium at the time when the culture started, and 6 and 9 hours later. The culture was carried out at 37° C at 120 spm by charging 5 ml each of Sabouraud dextrose medium in a large test tube with cotton stopper.

As the result a tendency that fungal growth was somewhat inhibited 6 and 9 hours after was observed, where AJI9463A, AJI9463B and AJI9463C and the partially hydrolyzed disaccharides thereof were added. In addition, the fungal count in one group was clearly increased and the morphological abnormality, i.e. that fission of the cells after budding of yeast was inhibited was observed. This morphological abnormality was markedly observed especially 6 hours after the start of culture.

(6) Cytotoxicity of AJI9463A, AJI9463B and AJI9463C and the partially hydrolyzed disaccharides thereof

Mouse ascites breast cancer cell FM3A was inoculated on Dulbecco's modified MEM medium supplemented with 10% calf fetal serum in 1 x $10^5$ counts/ml and AJI9463A, AJI9463B AJI9463C and each of the partially hydrolyzed disaccharides thereof were added to the medium in 1 mg each/ml followed by stationary culture at 37° C for 4 days. The cultured mouse ascites breast cancer cell FM3A was microscopically observed but no growth inhibition was noted at all.

Human leukemia cell K562 was inoculated on RPMI 1640 medium supplemented with 10% calf fetal serum in 1 x $10^5$ counts/ml and AJI9463A, AJI9463B and each of AJI9463C and the partially hydrolyzed disaccharides thereof was added to the medium in 1 mg each/ml followed by stationary culture at 37° C for 4 days. The cultured human leukemia cell K562 was microscopically observed but no growth inhibition was noted at all.

The allosamidin compounds, AJI9463A, AJI9463B and AJI9463C of the present invention exhibit an extremely potent inhibitory activity against the chitinase derived from pathogenic fungi Candida albicans. Therefore the present invention can provide antifungal agents and chitinase inhibitors having an excellent effect. These compounds are produced by fermentation and thus readily mass-produced. Furthermore, AJI9463B and AJI9463C are considered to advantageous in mass production from a standpoint of chemical synthesis since these compounds have as the constituent component N-acetylglucosamine present in large quantities in the natural world. On the other hand, the compounds of chemical structure 2 have low molecular weights but exhibit an extremely potent inhibitory activity against the chitinase from Candida albicans. Therefore, the present invention can provide antifungal agents and chitinase inhibitors having an excellent effect which can be readily mass produced from an industrial view point.

## Claims

1. Allosamidin compounds AJI9463A, AJI9463B and AJI9463C represented by the following chemical structure :

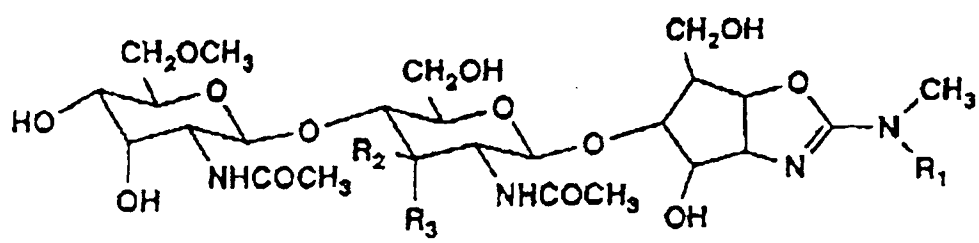

wherein in AJI9463A, $R_1$ and $R_2$ are hydrogen and $R_3$ is hydroxy; in AJI9463B $R_1$ and $R_3$ are hydrogen and $R_2$ is hydroxy; and in AJI9463C $R_1$ is methyl, $R_2$ is hydroxy and $R_3$ is hydrogen.

2. Allosamidin compounds represented by the following chemical structure :

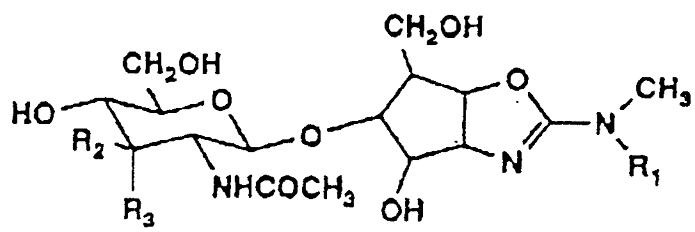

wherein $R_1$ represents hydrogen or methyl, and when $R_1$ is hydrogen, either $R_2$ or $R_3$ is hydrogen and another is hydroxy; when $R_1$ is methyl, $R_2$ is hydroxy and $R_3$ is hydrogen.

3. A process for preparing compounds as claimed in claim 1, which comprises culturing a microorganism belonging to the genus Streptomyces and capable of producing compounds as claimed in claim 1 and harvesting said compounds from the culture broth.

4. Use of the compounds as claimed in claim 1 or 2 as antifungal agents and chitinase inhibitors.

5. Pharmaceutical composition comprising the compounds as claimed in claim 1 or 2 as active components.

FIG. 1

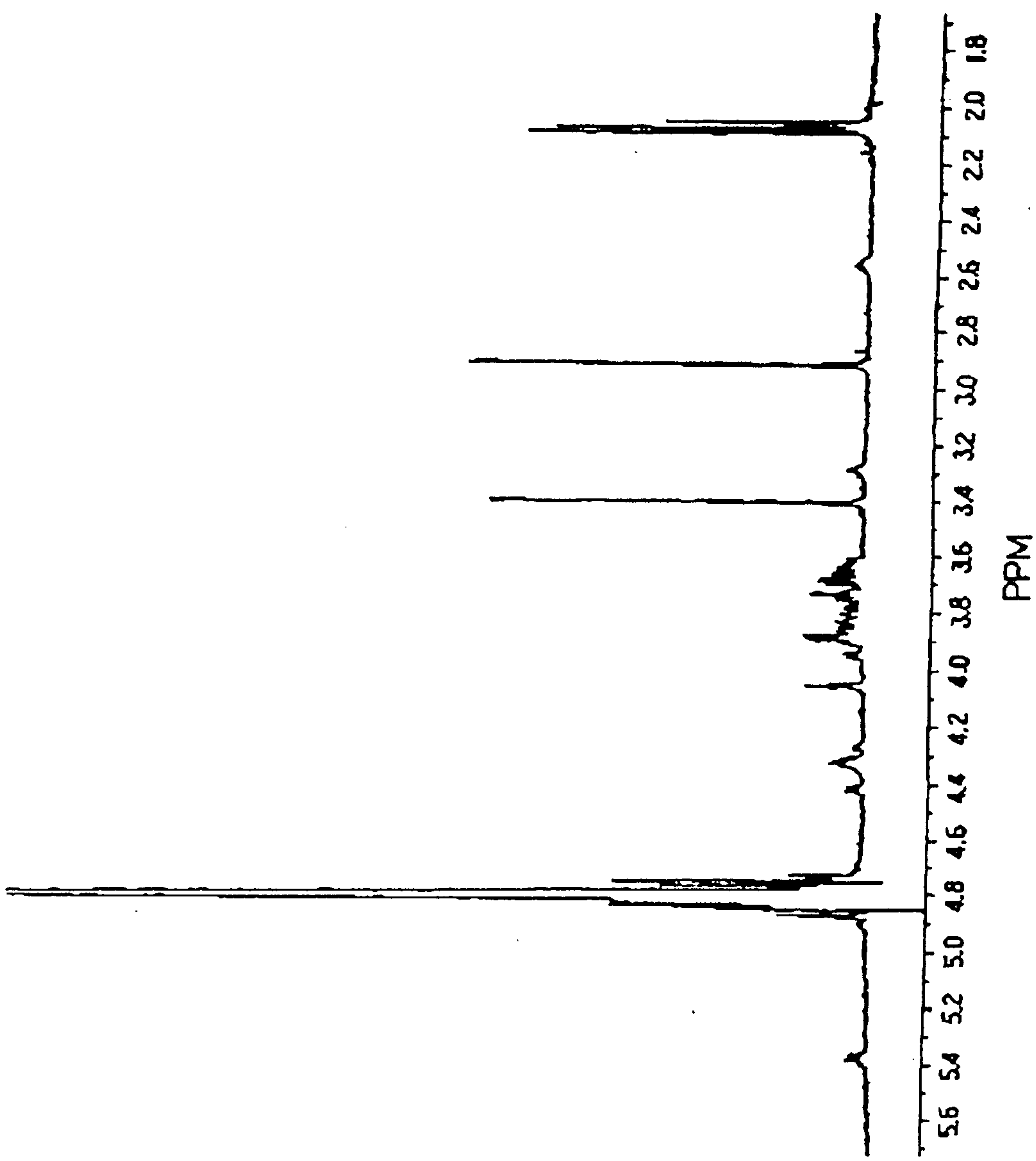

FIG. 2

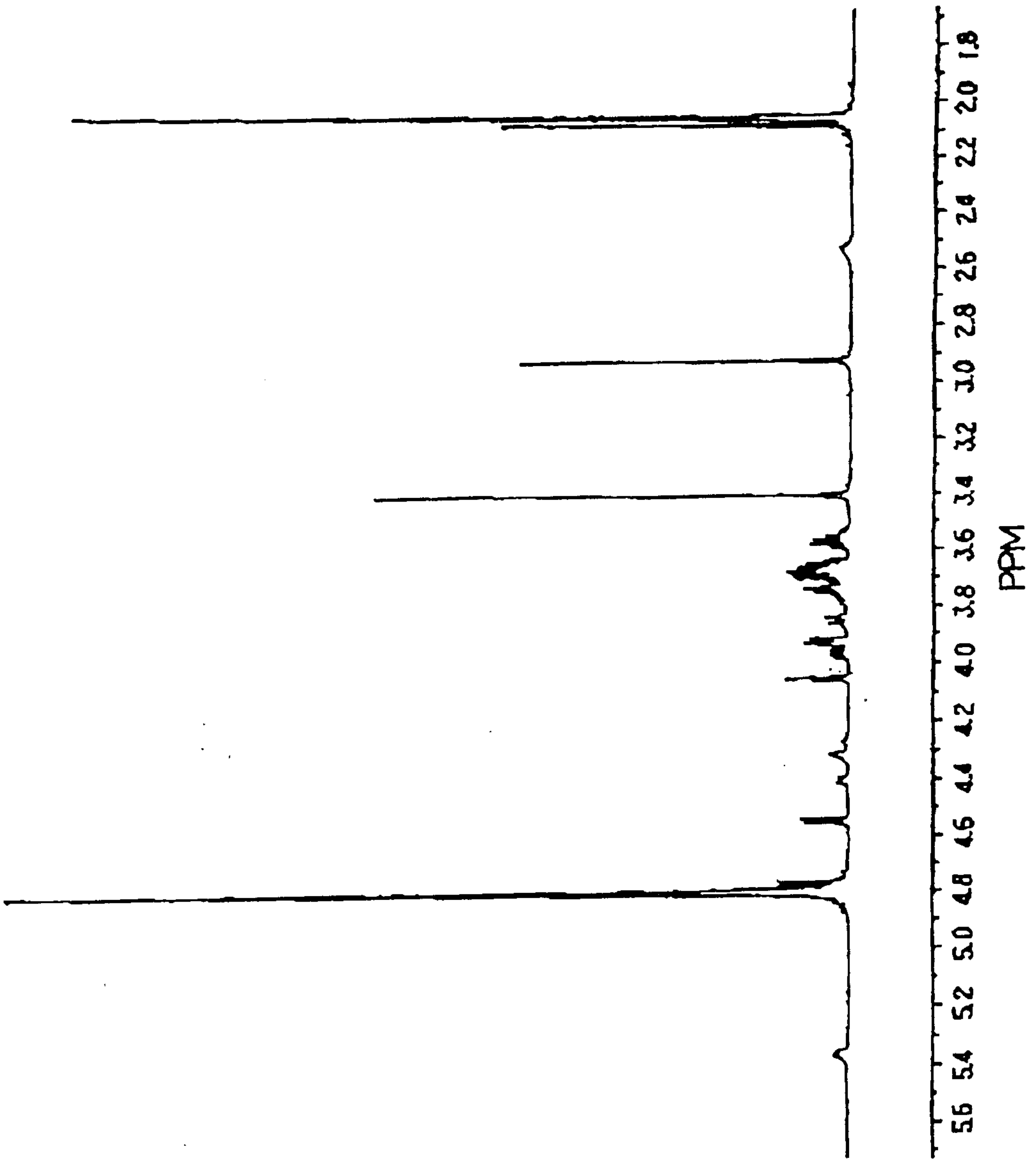

FIG. 3

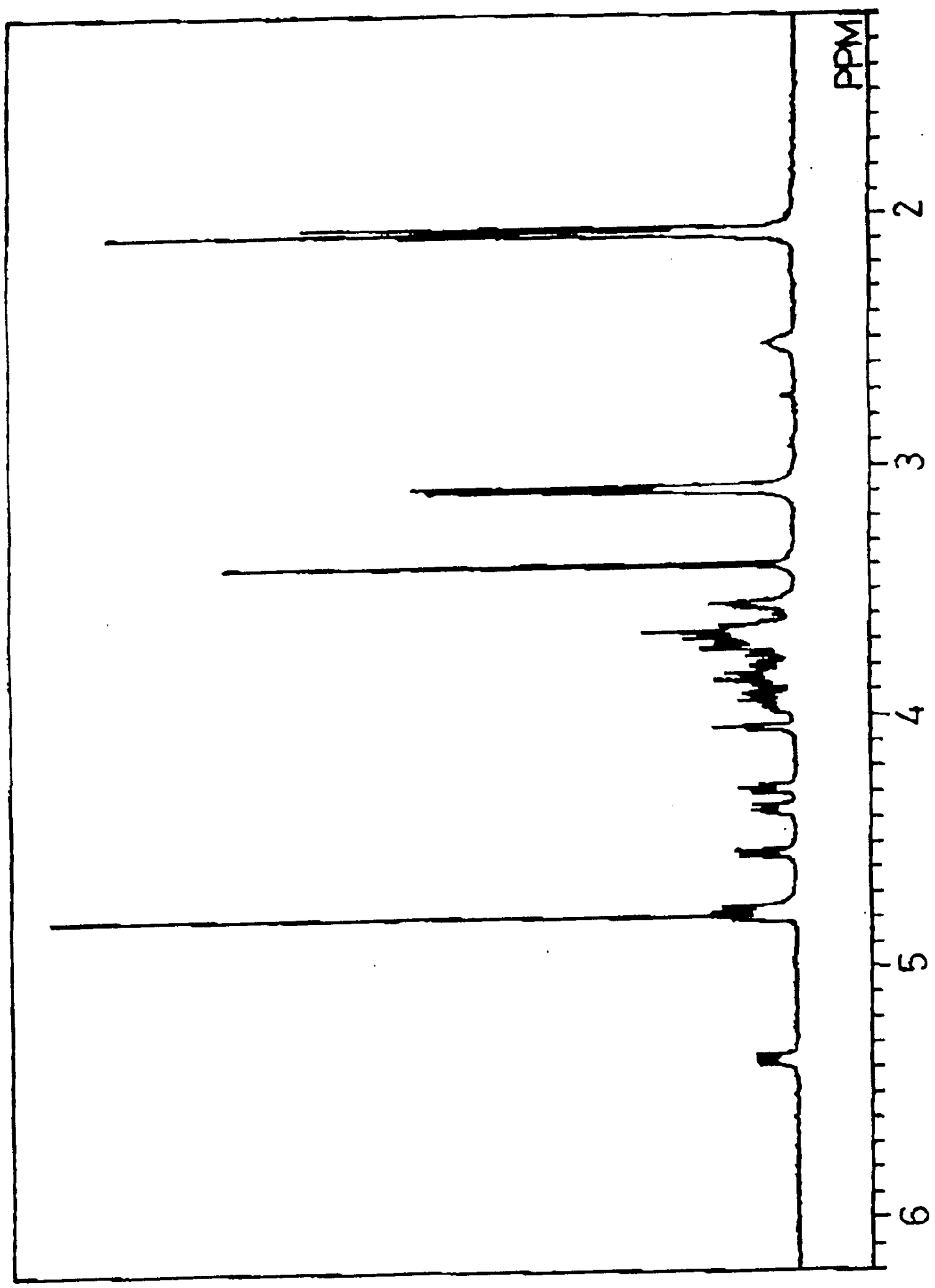

FIG. 4

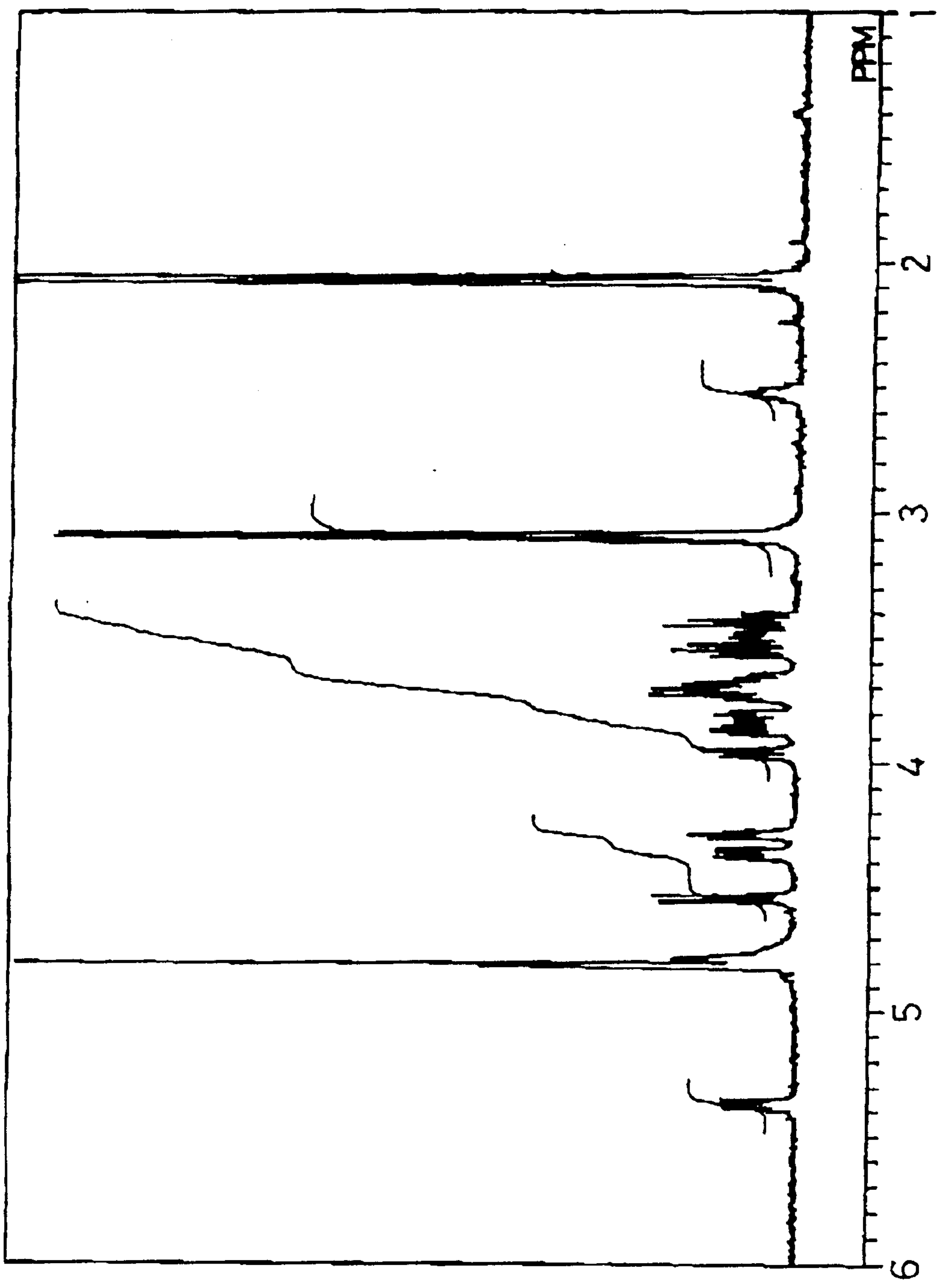

European Patent Office

# EUROPEAN SEARCH REPORT

Application Number

EP 92 10 1468
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | JOURNAL OF ANTIBIOTICS.<br>vol. 44, no. 7, July 1991, TOKYO JP<br>pages 716 - 722;<br>Y.NISHIMOTO ET AL.: 'Isolation and Characterization of New Allosamidins'<br>* the whole document *<br>*page 719, table, especially compounds (4)-(6)*<br>--- | 1-5 | C07H17/00<br>A01N43/76<br>A61K31/70<br>C12P19/60 |
| Y | AGRICULTURAL AND BIOLOGICAL CHEMISTRY.<br>vol. 54, no. 4, April 1990, TOKYO JP<br>pages 1333 - 1335;<br>S.SAKUDA ET AL.: 'Effects of Demethylallosamidin, a Potent Yeast Chitinase Inhibitor, on the Cell division of Yeast'<br>* the whole document *<br>--- | 1-5 | |
| Y | AGRICULTURAL AND BIOLOGICAL CHEMISTRY.<br>vol. 53, no. 10, October 1989, TOKYO JP<br>pages 2825 - 2826;<br>A.ISOGAI ET AL.: 'Structure of Demethylallosamidin as an Insect Chitinase Inhibitor'<br>* the whole document *<br>--- | 1-5 | TECHNICAL FIELDS SEARCHED (Int. Cl.5) |
| Y,D | AGRICULTURAL AND BIOLOGICAL CHEMISTRY.<br>vol. 51, no. 12, December 1987, TOKYO JP<br>pages 3251 - 3259;<br>S.SAKUDA ET AL.: 'Structures of Allosamidins, Novel Insect Chitinase Inhibitors, Produced by Actinomycetes'<br>* the whole document *<br>--- | 1-5 | C07H<br>A01N<br>A61K<br>C12P |
| Y | EP-A-0 395 106 (AJINOMOTO CO. INC.)<br>* the whole document *<br>---<br>-/-- | 1-5 | |

The present search report has been drawn up for all claims

| Place of search | Date of completion of the search | Examiner |
|---|---|---|
| THE HAGUE | 17 MARCH 1992 | SCOTT J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 92 10 1468
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | PATENT ABSTRACTS OF JAPAN vol. 12, no. 70 (C-479)(2917) 4 March 1988 & JP-A-62 207 294 ( SANKYO CO. LTD. ) 11 September 1987 * abstract * ----- | 1-5 | |
| | | | TECHNICAL FIELDS SEARCHED (Int. Cl.5 ) |

The present search report has been drawn up for all claims

| Place of search | Date of completion of the search | Examiner |
|---|---|---|
| THE HAGUE | 17 MARCH 1992 | SCOTT J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document